# EUROPEAN PATENT APPLICATION

(11) **EP 4 170 038 A1**
(43) Date of publication of application: **26.04.2023**
(21) Application number: 21828118.6
(22) Date of filing: 10.06.2021
(51) Int. Cl.: C12N 15/81, C12N 15/10, C12P 13/02

(54) **PICHIA CIFERRII MUTANT STRAIN HAVING IMPROVED SPHINGOLIPID AND SPHINGOID BASE PRODUCTIVITY, AND PREPARATION METHOD THEREFOR**

(30) Priority: 23.06.2020 KR 20200076795
(71) Applicant: Solus Biotech Co., Ltd., Iksan-si, Jeollabuk-do 54588 (KR)
(72) Inventor: AHN, Jung Oh, Daejeon 34141 (KR); HAN, Chang Pyo, Daejeon 34141 (KR); JANG, Min Jeong, Daejeon 34141 (KR); KWON, Hee Un, Daejeon 34141 (KR); LEE, Tae Hee, Seongnam-si, Gyeonggi-do 13597 (KR); LEE, Ju Hee, Hwaseong-si, Gyeonggi-do 18477 (KR); KIM, Hye Jin, Anyang-si, Gyeonggi-do 14051 (KR)
(74) Representative: dompatent von Kreisler Selting Werner - Partnerschaft von Patent- und Rechtsanwälten mbB
(86) International application number: PCT/KR2021/007298
(87) International publication number: WO 2021/261816

(57) **Abstract**

The present disclosure relates to a novel yeast mutant strain for producing a ceramide precursor, and a method for preparing the same, and more specifically, to a *Pichia ciferrii* mutant strain genetically engineered to overexpress sphingolipid metabolites prepared through the reconstruction of metabolic pathways for ceramide production in *Pichia ciferrii,* or a method for preparing the mutant strain.

## Description

### [Technical Field]

This application claims the benefit of Korean Patent Application No. 10-2020-0076795 filed on June 23, 2020, with the Korean Intellectual Property Office, the disclosure of which is herein incorporated by reference in its entirety.

The present disclosure relates to a *Pichia ciferrii* mutant strain with improved productivity of sphingolipids and sphingoid bases by regulating fatty acid metabolism, and a method for preparing the same.

### [Background Art]

Sphingolipid refers to a group of lipids derived from sphingoid bases, and is a common name for ceramides or ceramide derivatives that have sphingosine, phytosphingosine, and sphinganine as their basic skeletons in the human body. Sphingolipid is naturally present in large amounts in the cell membranes of animals, plants and microorganisms, and is involved in various cell signaling such as cell differentiation, cell growth and cell death.

It is known that ceramides have both hydrophilic and lipophilic groups to prevent evaporation of moisture from the skin, as well as to act as a defense substance with permeability to the stratum corneum of human skin and homeostasis against microorganisms. Recently, ceramides have been used as useful substances in cosmetics.

Tetraacetyl phytosphingosine (TAPS), which is valuable as a precursor of ceramide, is a substance produced in a completely acetylated state by N, O-acetylation of phytosphingosine in sphingoid bases, and is easily converted back to phytosphingosine through a deacetylation reaction. The chemical structure of the converted phytosphingosine has the same D-erythro isomer structure as the ceramide structure of human skin, and thus, it may be used as a raw material for producing ceramide friendly to human skin.

TAPS may be chemically synthesized (Current Organic Chemistry, 2010. 14(20) 2483-2521), but the process is complicated and the production cost is high. Therefore, as a method for replacing it from an economic point of view, production through biological methods is attracting attention. It was found that among them, *Pichia ciferrii* produced a large amount of sphingoid bases and sphingolipids including TAPS (Journal of bacteriology, 1960, 80(4) 484-491), and afterwards, a brief process for the biosynthesis of TAPS was known through genome sequencing (Eukaryotic cell, 2012, 11(12) 1582).

Wild-type *Pichia ciferrii* was known to produce 233 mg/L of TAPS at a yield of 5 mg (TAPS)/g (glucose) in an experiment conducted for research purposes (Applied Microbiology, 1962, 10(5) 401). In addition, the newly isolated *Pichia ciferrii* DSCC 7-25 showed a TAPS production yield of 1,065 mg/L through the method of adjusting culture conditions and separating and purifying sphingolipids (Korean Patent No. 10-0221357, Korean Patent No. No. 10-0188857). However, this is nothing more than a production yield improvement at the laboratory level, and does not reach the level of mass production for industrial use.

Therefore, the present disclosure is intended to provide a *Pichia ciferrii* mutant strain with increased production yield of TAPS to enable industrial utilization and a method for producing the same.

### [Prior Art References]

### Patent Documents

Korean Patent No. 10-0221357
Korean Patent No. 10-0188857
Korean Patent No. 10-2056651

### Non-Patent Documents

Current Organic Chemistry, 2010. 14(20) 2483-2521 Journal of bacteriology, 1960, 80(4) 484-491
Eukaryotic cell, 2012, 11(12) 1582
Applied Microbiology, 1962, 10(5) 401
Science 2001, 292(5516) 504
Biochimica et Biophysica Acta(BBA) -Lipids and Lipid Metabolism, 1973. 306(2) 341-345

### [Disclosure]

### [Technical Problem]

It is an object of the present disclosure to provide a genetically engineered *Pichia ciferrii* mutant strain with increased productivity of sphingolipids and sphingoid bases and a method for preparing the same.

Additionally, it is another object of the present disclosure to provide a method for producing TAPS, which is a ceramide precursor, using the genetically engineered *Pichia ciferrii* mutant strain.

### [Technical Solution]

In order to achieve the above objects, the present disclosure provides a genetically engineered *Pichia ciferrii* mutant strain.

According to an embodiment of the present disclosure, there is provided a *Pichia ciferrii* mutant strain in which the expression of a gene related to β-oxidation of palmitoyl-CoA is suppressed to increase palmitoyl-CoA accumulation in the cell.

According to an embodiment of the present disclosure, there is provided a *Pichia ciferrii* mutant strain in which the PcPOX3 gene, a gene related to β-oxidation of palmitoyl-CoA, is deleted to suppress its expression.

According to an embodiment of the present disclosure, there is provided a *Pichia ciferrii* mutant strain in which the expression of the PcLCB4 gene, which is a gene involved in phosphorylation of sphingoid bases, is further suppressed in the PcPOX3 gene-deleted *Pichia ciferrii* cell.

According to an embodiment of the present disclosure, there are provided a culture method and a production method of tetraacetyl phytosphingosine (TAPS), for increasing the production of TAPS, which is a precursor of ceramide, in the cell.

According to an embodiment of the present disclosure, there is provided a method for producing TAPS, which is a precursor of ceramide, in the *Pichia ciferrii* mutant strain.

### [Advantageous Effects]

The *Pichia ciferrii* mutant strain provided by the present disclosure provides a genetically engineered *Pichia ciferrii* strain with increased productivity of sphingolipids and sphingoid bases compared to its wild type. In addition, the present disclosure provides an environmentally friendly production method capable of producing sphingolipids and sphingoid bases through biological methods.

### [Description of Drawings]

Figure 1 shows the construction diagram of the auxotrophic PcURA3 gene deletion cassette vector.
Figure 2 shows the principle of construction and continuous deletion of a deletion cassette vector related to fatty acid metabolism.
Figure 3 is a graph showing TAPS production of each *Pichia ciferrii* mutant strain.

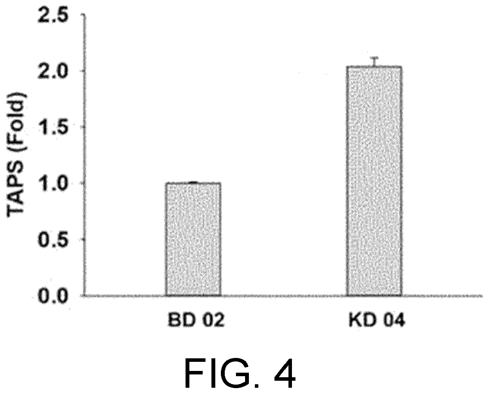

Figure 4 is a graph showing TAPS production of the PcPOX3 gene-deleted *Pichia ciferrii* mutant strain in a medium containing C12 FAME.

### [Best Mode]

Hereinafter, the present disclosure will be described in more detail in order to facilitate understanding of the present disclosure.

*Pichia ciferrii* is the only yeast with the ability to produce and secrete a large amount of fully acetylated sphingolipids, which cannot be found in other yeasts (Journal of bacteriology, 1960, 80(4) 484-491), and the exact origin of evolution to this phenotype has not yet been elucidated, but it is assumed that it evolved through competition to secure a carbon energy source in amino acid mass-producing yeasts or Crabtree-positive strains (Science 2001, 292(5516) 504).

*Pichia siferri* is an unconventional yeast strain, which was formerly named *Hansenula ciferrii* or *Pichia ciferrii,* but now reclassified to the genus *Wickerhamomyces.* However, in the present disclosure, in order to prevent confusion with the existing name, it was specified as *Pichia ciferrii* as per the existing name. The main feature of *Pichia ciferrii* is that it is the only yeast with the ability to produce and secrete a large amount of fully acetylated sphingoids, which cannot be found in other yeasts, and may produce and secrete TAPS, which is valuable as a precursor for cosmetic ceramides as described above.

The first step in the biosynthetic process of sphingolipids is the step of condensing serine and palmitoyl-CoA by serine palmitoyl transferase (3-ketosphinganine synthase) to form 3-ketosphinganine having 18 carbon atoms, and this step determines the rate of sphingolipid biosynthesis (Biochimica et Biophysica Acta (BBA) -Lipids and Lipid Metabolism, 1973. 306(2) 341-345).

There is a metabolic process in which palmitoyl-CoA, which is a raw material for TAPS biosynthesis together with serine (L-serine), is decomposed into acetyl-CoA by the β-Oxidation process. Blocking this metabolic process may enhance the conversion of palmitic acid to palmitoyl-CoA, thereby inducing palmitoyl-CoA accumulation and increasing TAPS production.

The gene related to the enzyme that catalyzes the reaction of converting palmitoyl-CoA to trans-Δ2-enoyl-CoA in the β-Oxidation process is known to be the POX gene, and similar genes named PcPOX3 and PcPOX4 in *Pichia ciferrii* are also known. The accumulation rate of palmitoyl-CoA may be increased by suppressing the expression of the corresponding genes.

In addition, in fatty acid metabolism, PcLCB4 (sphinganine kinase LCB4) is an enzyme having a kinase activity that phosphorylates sphinganine and phytosphingosine, it is known that deletion of the PcLCB4 gene suppresses metabolism toward sphingosine, thereby increasing TAPS production (Korean Patent No. 10-2056651).

The present disclosure relates to a *Pichia ciferrii* mutant strain in which the expression of the PcPOX3 gene (SEQ ID NO: 28) or the PcPOX3 (SEQ ID NO: 28) and PcLCB4 (SEQ ID NO: 29) genes is suppressed, and a method for suppressing the expression of the genes includes partial or total deletion of each gene, insertion of foreign DNA, replacement of a part of a gene with a foreign gene, RNA interference, induction of gene mutation, or a combination thereof, specifically, is by partial or total deletion of each gene, and more specifically, is by total deletion of each gene.

In the present disclosure, the accumulation rate of palmitoyl-CoA may be increased by constructing a deletion cassette targeting the corresponding genes, and in the present disclosure, the PcPOX3 gene-deleted *Pichia ciferrii* mutant strain and the PcPOX3 and PcLCB4 genes-co-deleted *Pichia ciferrii* mutant strain were prepared and increased TAPS production was confirmed.

It is understood that the reduction in enzyme activity is compared to a wild-type *Pichia ciferrii* strain.

The "wild-type" of a cell in the context of the present disclosure preferably refers to the parental strain from which the cell according to the present disclosure is developed through modification of elements affecting the activity of an enzyme encoded by a specified nucleic acid sequence (for example, a gene comprising a specified nucleic acid sequence encoding the corresponding enzyme, or a promoter present in the corresponding gene and functionally linked to the specified nucleic acid sequence).

The "reduction in enzyme activity" compared to wild-type preferably refers to an activity that is reduced by at least 50%, particularly preferably by at least 90%, further preferably by at least 99.9%, further more preferably by at least 99.99%, and most preferably by at least 99.999% compared to the wild-type activity.

The reduction in specific activity of the cells according to the present disclosure compared to its wild-type may be measured by, but not limited to, a method for measuring activity by using cells grown under the same conditions, such as equivalent cell numbers/concentrations, for example, media, gas treatment, and agitation, if possible.

The yeast mutant strain of the present disclosure is a mutant strain in which a sphingolipid metabolic pathway is redesigned using a wild-type *Pichia ciferrii* strain as a parent strain, and preferably, the *Pichia ciferrii* DSCC 7-25 (Accession No. KFCC-10937) strain disclosed in the Examples of Korean Patent No. 10-0188857 may be used, or any strain having the sphingolipid metabolic pathway may be used as a parent strain without any limitation to the strain.

In an embodiment of the present disclosure, TAPS production (TAPS-specific yield) in the mutant strain through the above method was about 1.7-fold in the PcPOX3 gene-deleted mutant strain, and 2-fold or more in the PcPOX3 and PcLCB4 genes-co-deleted mutant strain.

An embodiment of the present disclosure provides a method for increasing TAPS production by adding fatty acid methyl esters to the culture medium of the mutant strain to artificially accumulate fatty acids in cells. As a result, the TAPS-specific yield increased to about 100% in the PcPOX3 gene-deleted mutant strain.

With respect to the above-mentioned sequences, "nucleotide identity" may be measured with the aid of known methods. In general, special computer programs are used with algorithms that take into account special requirements.

It is another object of the present disclosure to provide the mutant strain according to the present disclosure that produces sphingoid bases and sphingolipids.

The term "sphingoid base" in the context of the present disclosure refers to one selected from phytosphingosine, sphingosine, sphingadienine, 6-hydroxysphingosine and sphinganine (dihydrosphingosine), and their acetylated forms, such as, for example, tetraacetylphytosphingosine, triacetylphytosphingosine, diacetylphytosphingosine, O-acetylphytosphingosine, triacetylsphinganine, diacetylsphinganine, O-acetylsphinganine, triacetylsphingosine, diacetylsphingosine, O-acetylsphingosine, tetraacetyl-6-hydroxysphingosine, triacetyl-6-hydroxysphingosine, diacetyl-6-hydroxysphingosine, O-acetyl-6-hydroxysphingadienine, triacetylsphingadienine, diacetylsphingadienine, and O-acetylsphingadienine.

The term "sphingolipid" in the context of the present disclosure is understood to refer to a compound comprising a sphingoid base covalently bonded to a fatty acid via an amide bond. The fatty acid may be saturated or mono- or polyunsaturated. The fatty acid side chains may vary in length. The fatty acid side chains may also contain functional groups such as hydroxyl groups. The sphingolipids include, for example, phytoceramides, ceramides and dihydroceramides, and the more complex glucosylceramides (cerebrosides) and inositol phosphorylceramides, mannosylinositol phosphorylceramides and mannosyldiinositol phosphorylceramides. In this case, the sphingolipids also include sphingoid bases bonded to an acetyl radical via an amide bond, such as, for example, N-acetylphytosphingosine, N-acetylsphinganine, N-acetylsphingosine and N-acetyl-6-hydroxysphingosine. These compounds are also known by the term short-chain ceramides.

The use of the mutant strain according to the present disclosure is advantageous for producing sphingolipids and sphingoid bases selected from the group consisting of phytosphingosine, sphingosine, sphingadienine, 6-hydroxysphingosine, sphinganine (dihydrosphingosine), tetraacetylphytosphingosine (TAPS), triacetylphytosphingosine, diacetylphytosphingosine, O-acetylphytosphingosine, N-acetylphytosphingosine, triacetylsphinganine (TriASa), diacetylsphinganine, O-acetylsphinganine, N-acetylsphinganine, triacetylsphingosine (TriASo), diacetylsphingosine, O-acetylsphingosine, N-acetylsphingosine, tetraacetyl-6-hydroxysphingosine, triacetyl-6-hydroxysphingosine, diacetyl-6-hydroxysphingosine, O-acetyl-6-hydroxysphingosine, N-acetyl-6-hydroxysphingosine, triacetylsphingadienine, diacetylsphingadienine, and O-acetylsphingadienine. The use of the mutant strain according to the present disclosure for producing tetraacetylphytosphingosine (TAPS) is preferred.

It is another object of the present disclosure to provide a method for producing the above-described mutant strain according to the present disclosure. The method comprises the steps of:
a) providing a *Pichia ciferrii* strain, and
b) modifying at least one gene involved in β-oxidation by insertion of foreign DNA or DNA encoding a selectable marker gene into the gene, deletion of at least a portion of a gene, point mutation in a gene sequence, exposure of a gene to the effects of RNA interference, and partial replacement of a gene by foreign DNA or foreign DNA in a promoter region.

The *Pichia ciferrii* mutant strain according to the present disclosure may be used to prepare sphingoid bases and sphingolipids, and is preferably suitable for, but is not limited to, improving the production yield of tetraacetylphytosphingosine (TAPS).

Hereinafter, a method for producing sphingoid bases and sphingolipids using the *Pichia ciferrii* mutant strain according to the present disclosure will be described in detail, comprising the steps of:
a) contacting the genetically modified mutant strain according to an embodiment with a medium containing a carbon source;
b) culturing the strain under conditions that allow the strain to produce sphingoid bases and sphingolipids from the carbon source; and
c) optionally, isolating the resulting sphingoids base and sphingolipids.

Hereinafter, an additional method for producing sphingoid bases and sphingolipids using the *Pichia ciferrii* mutant strain according to the present disclosure will be described in detail, comprising the steps of:
a) contacting the genetically modified mutant strain according to an embodiment with a medium containing a carbon source and fatty acid methyl ester (FAME);
b) culturing the mutant strain under conditions that allow the mutant strain to produce sphingoid bases and sphingolipids from the carbon source; and
c) optionally, isolating the resulting sphingoids base and sphingolipids.

The carbon source that may be used in the step of culturing the cell includes carbohydrates, such as, for example, glucose, fructose, glycerol, sucrose, maltose, molasses, or other alcohols, such as, for example, ethanol, and organic acids, such as, for example, acetate. The nitrogen source that may be used includes, for example, ammonia, ammonium sulfate, ammonium nitrate, ammonium chloride, organic nitrogen compounds (for example, yeast extract, malt extract, peptone, corn steep liquor). Inorganic compounds, such as, for example, phosphates, magnesium salts, potassium salts, zinc salts, iron salts and the like may also be used.

Suitable culture conditions for the *Pichia ciferrii* strain of the present disclosure are known to those skilled in the art, for example, from International Publication No. WO 2007/131720 or Korean Patent No. 10-0221357.

Hereinafter, the present disclosure will be described in detail by way of examples.

However, the following examples are only for illustrating the present disclosure, and the content of the present disclosure is not limited to the following examples. The scope of use of the disclosure follows the entire detailed description and claims.

### [Best Mode]

### <Example>

### <Example 1> Construction of an auxotrophic gene deletion cassette

Unless otherwise specified, the subsequent construction of the cassette was constructed by *E. coli* plasmid cloning. Gene deletion was performed by replacing DNA by classical homologous recombination and double cassette utilization (Yeast, 1996. 12(14): 1439-1457).

The present inventors first discovered and deleted the auxotrophic gene PcURA3 for efficient utilization of the marker gene, and then used it as a marker for deletion of the PcPOX3 and PcLCB4 genes (Example 2).

Specifically, a PcURA3 deletion cassette was constructed using the plasmid pGEM-T-easy (Promega) vector as a transfer vector. The pGEM-T-easy vector was cleaved using restriction enzymes SpeI and SacI, and the resulting 2.97 kb DNA fragment was used as a backbone for subsequent cloning.

The 600 bp sequence at the 5'-end of the GAPDH gene (SEQ ID NO: 1) isolated from the genome of wild-type *Pichia ciferrii* cells and the DNA-synthesized antibiotic nourseothricin resistance gene PcNAT1 (SEQ ID NO: 2) were amplified by PCR amplification.

The 5'-end (P-GAP1) of the PcGAPDH gene (SEQ ID NO: 1) was cleaved using restriction enzymes SpeI and NdeI, and the two DNA fragments of the PcNAT1 gene (SEQ ID NO: 2) cleaved using SacI and NdeI were ligated with the previously cleaved pGEM-T-easy vector fragment through T4 DNA ligase enzyme (TAKARA) treatment.

The GAPDH ends ligated on the vector were cleaved using restriction enzymes SpeI and EcoRI, and the resulting 4.147 kb DNA fragment was used as a backbone for subsequent cloning. The 150 bp sequence T-PcTEF at the 3'-end of the PcTEF1 gene isolated from the genome of wild-type *Pichia ciferrii* cells was amplified by PCR amplification (SEQ ID NO: 27).

The T-PcTEF was cleaved using restriction enzymes SpeI and EcoRI, and ligated with the GAPDH end of the previously cleaved pGEM-T-easy vector through T4 DNA ligase enzyme (TAKARA) treatment.

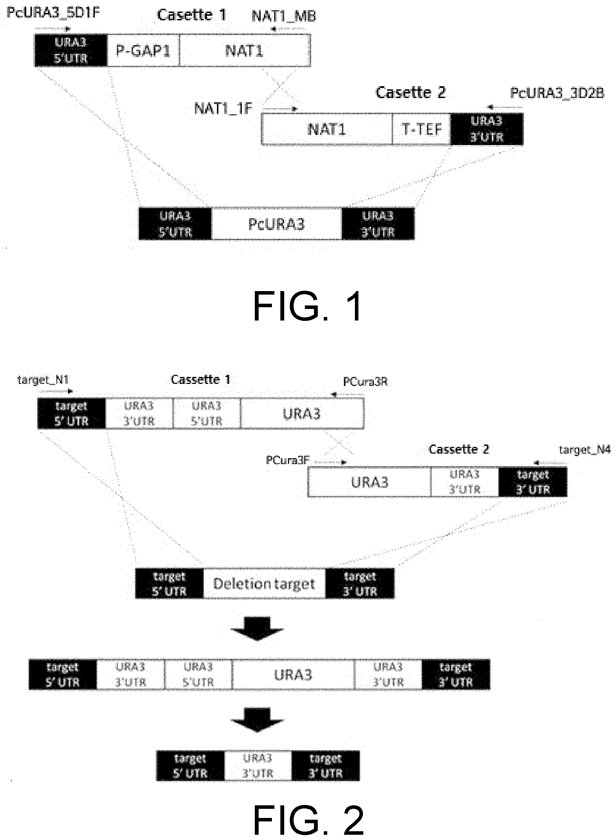

The GAPDH end (Figure 1, P-GAP1) ligated on the vector, 1335 bp of DNA of PcNAT1 gene and PcTEF end (Figure 1, T-PcTEF), 1171 bp of the 5'-end and 548 bp of 3'-end of PcURA3 gene (SEQ ID NO: 3) isolated from the genome of wild-type *Pichia ciferrii* cells were amplified by PCR. At this time, the PcGAPDH end and the 5'-end of the PcURA3 gene, and the PcTEF gene end and the 3'-end of the PcURA3 gene were overlapped by 22 bp and 25 bp.

Cassette 1 in Figure 1 was amplified by PCR using the primer NAT1_MB (cacctgaatcaggatattcttctaatacctgg; SEQ ID NO: 9) located at 324 bp from the initiation codon of the PcNAT1 gene and the primer PcURA3_5D1F (tgagaaacgtggcaatggatcattg; SEQ ID NO: 10) located at 1171 bp of the 5'-end of the PcURA3 gene (SEQ ID NO: 3).

Cassette 2 in Figure 1 was amplified by PCR using the primer NAT1_1F (atgggtaaggaaaagactcacgttt; SEQ ID NO: 11) located at the initiation codon of the PcNAT1 gene and the primer PcURA3_3D2B (ttgtcactcttgaatggcatctactg; SEQ ID NO: 12) located at 548 bp of the 3'-end of the PcURA3 gene.

All primers described were marked in the 5' to 3' direction.

The PcURA3 gene (SEQ ID NO: 3) was deleted by transforming the deletion cassette (SEQ ID NO: 4) prepared by the method of Example 1 into a *Pichia ciferrii* strain, and the inserted cassette was confirmed through PCR and DNA sequencing (Figure 1) .

### <Example 2> Construction of gene deletion cassettes related to fatty acid and sphingolipid metabolism

The principle of construction of the basic cassette vector was the same as that of the auxotrophic gene deletion cassette.

In this example, the target gene to be constructed by a deletion cassette was PcPOX3, PcLCB4, or both PcPOX3 and PcLCB4. The deletion cassette was constructed using the plasmid pGEM-T-easy (Promega) as a transfer vector. The pGEM-T-easy (Promega) vector was cleaved using restriction enzymes NotI and SpeI, and the resulting 3.73 kb DNA fragment was used as a backbone for subsequent cloning.

Using the genome of wild-type *Pichia ciferrii* as a template, the ORF of the PcURA3 gene from 359 bp of the 5'-end to 387 bp of the 3'-end were amplified by PCR so as to overlap 30 bp with the SpeI-cleaved section of the pGEM-T-easy vector fragment described above.

Using the genome of wild-type *Pichia ciferrii* as a template, the 387 bp of the 3'-end of the PcURA3 gene was amplified by PCR so that the 5'-end overlapped 30 bp with the NotI-cleaved section of the previously described pGEM-T-easy vector fragment and the 3'-end overlapped 30 bp from 329 bp to 359 bp of the 5'-end of the URA3 gene.

The two DNA fragments previously amplified by PCR and the cleaved pGEM-T-easy vector fragment were made to have repetitive sequences at both ends of the PcURA3 gene through infusion cloning (TAKARA).

The 5'-end site and 3'-end site (Table 1) of the target gene to be deleted were amplified by PCR, respectively, and at this time, the 5'-end of the primer was extended to overlap 30-35 bp with the repetitive sequences of the marker gene to be fused (Table 1).

**[Table 1]**

| List of corresponding primer sequences of genes to be deleted | | | | | | |
|---|---|---|---|---|---|---|
| Gene | Corresponding primer sequences of the target gene to be deleted | | | | | |

| name | N1 | N2 | N3 | N4 | N5 | N6 |
|---|---|---|---|---|---|---|
| PcPOX 3 | gatgcatgc tcgagcAGT ATCAAATCA ATTACTTAT ACACTTATG ACTTTTATG (SEQ ID NO: 15) | AGTATCAAA TCAATTACT TATACACTT ATGACTTTT ATG (SEQ ID NO: 16) | TATATTGTT GACCgcATA ACCAGCTAA TTTCACAAT TTTTTTGAA AGCT (SEQ ID NO: 17) | | | |
| | | | | cactggcgg ccgttaTTT CCATTACAT CAGAAAACA TTTCACAAT (SEQ ID NO: 18) | ccgagctcg gatccaATA TTTCCTTAA TATCCTTCT AACTTCTTC TTCA (SEQ ID NO: 19) | ATATTTCCT TAATATCCT TCTAACTTC TTCTTCA (SEQ ID NO: 20) |
| PcLCB 4 | gatgcatgc tcgagcAAA CTTATTCTC TTGTTTGGA ACAC (SEQ ID NO: 21) | AAACTTATT CTCTTGTTT GGAACAC (SEQ ID NO: 22) | TATATTGTT GACCgcTTC AAATCTTGA AGGACCTAA CC (SEQ ID NO: 23) | | | |
| | | | | cactggcgg ccgttaCGT ATGGCACCA ACTTTATTA TC (SEQ ID NO: 24) | ccgagctcg gatccaGCA ATTCTTGTT AAAGCAGCT C (SEQ ID NO: 25) | GCAATTCTT GTTAAAGCA GCTC (SEQ ID NO: 26) |

The previously constructed vector was ligated with the DNA fragment cleaved using restriction enzyme NotI and the 5'-end amplified by PCR using primers N1 and N3 of the target gene to be deleted (Figure 1), through infusion cloning (TAKARA).

The previously constructed vector was ligated with the DNA fragment cleaved using restriction enzyme SpeI and the 3'-end amplified by PCR using primers N4 and N5 of the target gene to be deleted, through infusion cloning (TAKARA).

Cassette 1 in Figure 2 was amplified by PCR using the primer PcURA3R (GCTCTGTAACGTTCACCTTC; SEQ ID NO: 13) located inside 43 bp from the stop codon of the PcURA3 gene and the reverse primer (N2) of the target terminal site to be deleted inserted at the 5'-end of the marker gene.

Cassette 2 in Figure 2 was amplified by PCR using the primer PcURA3F (ATTAGGTCCATATATCTGTCTTGTC; SEQ ID NO: 14) linked inside 150 bp gene from the initiation codon of the PcURA3 gene and the reverse primer (N6) of the target terminal site to be deleted inserted at the 3'-end of the marker gene.

All primers described were marked in the 5' to 3' direction.

Through this method, a specific gene deletion cassette having a PcPOX3 sequence (SEQ ID NO: 5, SEQ ID NO: 6) or a PcLCB4 sequence (SEQ ID NO: 7, SEQ ID NO: 8) was constructed.

The target gene was deleted by transforming the gene deletion cassette (SEQ ID NOs: 5 to 8) prepared by the method of Example 2 into a *Pichia ciferrii* strain, and the inserted cassette was confirmed through PCR and DNA sequencing.

For continuous gene deletion, *Pichia ciferrii* cells in which the marker gene was culled through recombination of repetitive sequences inserted at the 5'- and 3'-ends of the marker gene were constructed by addition of 5'-fluoroorotic acid and then confirmed by PCR.

### <Example 3> Selection of transformants

The constructed cassette was subjected to heat shock at a temperature of 37°C for 15 minutes, and transformed into the parent strain DSCC 7-25 and strains derived therefrom, and it was confirmed by PCR amplification whether or not the target gene was expressed, and a desired transformant was selected.

### <Example 4> TAPS production of transformants

Each of PcURA3 gene-deleted mutant strain in *Pichia ciferrii* DSCC 7-25 (Accession No. KFCC-1093) strain (BD 02), PcLCB4 gene-deleted mutant strain in the strain BD 02 (KD 02), PcPOX3 gene-deleted mutant strain in the strain BD 02 (KD 04), and PcLCB4 and PcPOX3 genes-co-deleted mutant strain in the strain BD 02 (KD 08) (Table 2) was inoculated into 50 ml of YGM optimal medium (glycerol 100 g/l, yeast extract 2 g/l, KNO₃ 3 g/l, (NH₄)₂SO₄ 0.5 g/l, MgSO₄·7H₂O 0.3 g/l, NaCl 0.5 g/l, CSL (corn steep liquor) 3 g/l, and LS-300 (anti-forming agent) 1 g/l) and cultured at 25°C for 5 days at 220 rpm, and then, 4 volumes of methanol were added thereto and reacted at room temperature for 1 hour, and then, the centrifuged supernatant was analyzed for TAPS.

TAPS was analyzed by HPLC using UV 200 nm. An 84.5:18.45:0.05 mixture of methanol, water and TFA (trifluoroacetic acid) was used as a solvent.

**[Table 2]**

| Characteristics of each mutant strain | |
|---|---|
| Strain name | Characteristics |
| BD 02 | Deletion of the PcURA3 gene in *Pichia ciferrii* DSCC 7-25 (Accession No. KFCC-10937) |
| KD 02 | Deletion of the PcLCB4 gene in strain BD 02 |
| KD 04 | Deletion of the PcPOX3 gene in strain BD 02 |
| KD 08 | Co-deletion of the PcPOX3 and PcLCB4 genes in strain BD 02 |

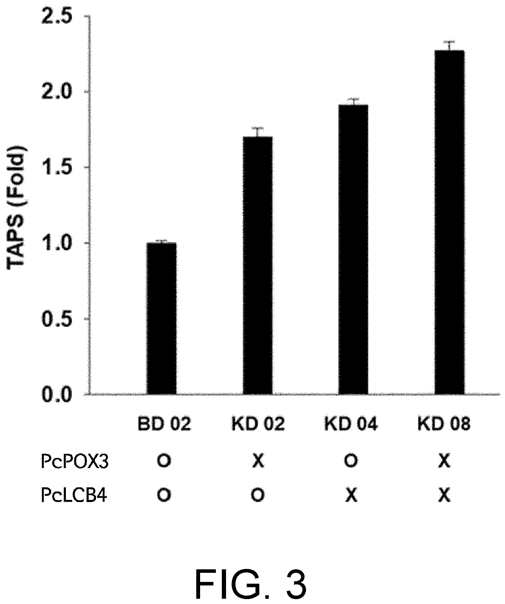

As shown in Table 3 below and FIG. 3, when the YGM optimal medium was used, the TAPS-specific yields of the PcLCB4 gene-deleted mutant strain (KD 02) and the PcPOX3 gene-deleted mutant strain (KD 04) relative to BD 02 increased 1.9-fold and 1.7-fold, respectively.

In addition, the PcLCB4 and PcPOX3 genes-co-deleted mutant strain (KD 08) relative to BD 02 showed a 2.3-fold higher TAPS-specific yield.

**[Table 3]**

| Results of analysis of TAPS production for each strain | | | | |
|---|---|---|---|---|
| | BD 02 | KD 02 | KD 04 | KD 08 |
| TAPS (mg/L) | 2460 | 3750 | 2800 | 3500 |
| TAPS-specific yield (mg/DCW) | 10.9 | 20.85 | 18.53 | 24.72 |
| Relative TAPS-specific yield (based on BD 02) | 1 | 1.9 | 1.7 | 2.3 |

### <Example 5> Addition of fatty acid methyl ester and production of TAPS in transformant strains

Each of the BD 02 and KD 04 transformant strains was inoculated into 50 ml of YGM optimal medium and cultured at 25°C for 3 days at 220 rpm, and then, 50 ml of fatty acid-containing medium (glycerol 30 g/l, yeast extract 2 g/l, KNO₃ 3 g/l, (NH₄)₂SO₄ 0.5 g/l, MgSO₄·7H₂O 0.3 g/l, NaCl 0.5 g/l, CSL 3 g/l, LS-300 1 g/l, lauric acid methyl ester 2.14 g/l, and Tween 80 0.5%) was added thereto and cultured at 25°C for 4 days at 220 rpm.

4 volumes of methanol was added thereto and reacted at room temperature for 1 hour, and then, the centrifuged supernatant was analyzed for TAPS.

TAPS was analyzed by HPLC using UV 200 nm. An 84.5:18.45:0.05 (w/w) mixture of methanol, water and TFA was used as a solvent.

As can be compared from the results in Table 4, when the optimal medium containing fatty acid methyl ester was used, the TAPS production of the PcPOX3-deleted mutant strain KD 04 relative to BD 02 increased by about 70% and the specific yield increased by 100% or more.

**[Table 4]**

| Results of analysis of TAPS production for each strain | | |
|---|---|---|
| | BD 02 | KD 04 |
| TAPS (mg/L) | 2240 | 3774 |
| TAPS-specific yield (mg/DCW) | 14.1 | 29 |
| Relative TAPS-specific yield (based on BD 02) | 1 | 2.1 |

## Claims

1. A *Pichia ciferrii* mutant strain genetically engineered to overexpress sphingolipid metabolites by intracellular palmitoyl-CoA accumulation induced by blockade of β-oxidation of palmitoyl-CoA in a wild-type *Pichia ciferrii* strain.

2. The *Pichia ciferrii* mutant strain according to claim 1, wherein the engineered gene is the PcPOX3 gene (SEQ ID NO: 28).

3. The *Pichia ciferrii* mutant strain according to claim 2, wherein expression of the gene is suppressed.

4. The *Pichia ciferrii* mutant strain according to claim 3, wherein a method for suppressing the expression of the gene comprises partial or total deletion of each gene, insertion of foreign DNA, replacement of a part of a gene with a foreign gene, RNA interference, induction of gene mutation, or a combination thereof.

5. The *Pichia ciferrii* mutant strain according to claim 4, wherein the method of suppressing the expression of the gene comprises partial or total deletion of each gene.

6. The *Pichia ciferrii* mutant strain according to claim 5, wherein expression of the PcLCB4 gene (SEQ ID NO: 29) is further suppressed.

7. The *Pichia ciferrii* mutant strain according to any one of claims 1 to 6, wherein the wild-type *Pichia ciferrii* cell is a *Pichia ciferrii* DSCC 7-25 (Accession No. KFCC-10937) strain.

8. A method for producing TAPS, comprising the steps of:
(a) culturing the *Pichia ciferrii* mutant strain according to any one of claims 1 to 6 in a medium containing a carbon source; and
(b) obtaining TAPS accumulated in the cultured strain.

9. A method for producing TAPS, comprising the steps of:
(a) culturing the *Pichia ciferrii* mutant strain according to any one of claims 1 to 6 in a medium containing fatty acid methyl ester (FAME); and
(b) obtaining TAPS accumulated in the cultured strain.

10. The method for producing TAPS according to claim 9, wherein the FAME is C12:0 methyl laurate.

11. A method for producing TAPS, comprising the steps of:
(a) culturing the *Pichia ciferrii* mutant strain according to claim 7 in a medium containing a carbon source; and
(b) obtaining TAPS accumulated in the cultured cell.

12. A method for producing TAPS, comprising the steps of:
(a) culturing the *Pichia ciferrii* mutant strain according to claims 7 in a medium containing fatty acid methyl ester (FAME); and
(b) obtaining TAPS accumulated in the cultured cell.

13. The method for producing TAPS according to claim 12, wherein the FAME is C12:0 methyl laurate.
